(19) Europäisches Patentamt
European Patent Office
Office européen des brevets

(11) Veröffentlichungsnummer: **0 022 749**
**B1**

(12) # EUROPÄISCHE PATENTSCHRIFT

(45) Veröffentlichungstag der Patentschrift: **18.05.83**

(21) Anmeldenummer: **80810221.4**

(22) Anmeldetag: **07.07.80**

(51) Int. Cl.³: **C 07 C 103/38,** C 07 C 103/78, C 07 D 209/48, C 07 D 209/78, C 07 D 251/34, C 07 D 487/04, C 08 K 5/20, C 08 K 5/34

(54) **Alkanolamid-beta-ketocarbonsäureester und ihre Verwendung zur Stabilisierung von chlorhaltigen Thermoplasten.**

(30) Priorität: **11.07.79 CH 6477/79**

(43) Veröffentlichungstag der Anmeldung: **21.01.81 Patentblatt 81/3**

(45) Bekanntmachung des Hinweises auf die Patenterteilung: **18.05.83 Patentblatt 83/20**

(84) Benannte Vertragsstaaten: **BE DE FR GB IT**

(56) Entgegenhaltungen:
**EP-A-0 002 616**
**DE-A-1 569 407**
**DE-A-2 852 785**

(73) Patentinhaber: **CIBA-GEIGY AG, Patentabteilung Postfach, CH-4002 Basel (CH)**

(72) Erfinder: **Wehner, Wolfgang, Dr., Wetzbach 34, D-6144 Zwingenberg (DE)**
Erfinder: **Michaelis, Klaus-Peter, Dr., Am Hangweg 8, D-6145 Lindenfels/Odenwald (DE)**
Erfinder: **Schneider, Rainer, Dr., Grafenstrasse 8, D-6140 Bensheim-Auerbach (DE)**

**EP 0 022 749 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Alkanolamid-β-ketocarbonsäureester und ihre Verwendung zur Stabilisierung von chlorhaltigen Thermoplasten

Die vorliegende Erfindung betrifft neue Alkanolamid-β-ketocarbonsäureester sowie ihre Verwendung allein oder mit anderen Stabilisatoren zur Stabilisierung von chlorhaltigen Thermoplasten.

Stickstofffreie Acetessigester sind als ungiftige Thermocostabilisatoren zur Stabilisierung von Polyvinylchloridharzen, die insbesondere für Nahrungs- und Genussmittelverpackung zur Anwendung kommen sollen, aus der DE-OS Nr. 1569407 bekannt. Es hat sich aber gezeigt, dass die stabilisierende Wirkung dieser Verbindungen in der Praxis nicht immer ganz befriedigende Resultate zeigt.

Es ist nun gefunden worden, dass Alkanolamid-β-ketocarbonsäureester eine überraschend gute, auch für die Praxis befriedigende Stabilisierung von chlorhaltigen Thermoplasten gewährleisten.

Demnach betrifft die vorliegende Erfindung Verbindungen der Formel I

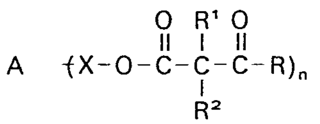 (I)

worin n die Zahlen 1 bis 3, R $C_1$ bis $C_{24}$-Alkyl oder Phenyl, $R^1$ und $R^2$ unabhängig voneinander Wasserstoff oder R und X gegebenenfalls durch $-O-$ unterbrochenes $C_1$ bis $C_6$-Alkylen bedeuten und, wenn n 1 ist, A eine der Gruppen der Formeln

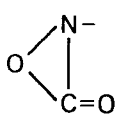 (II)

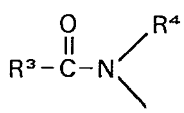 (III)

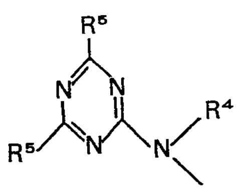 (IV)

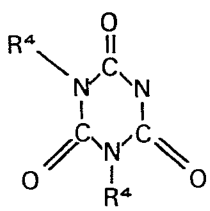 (V)

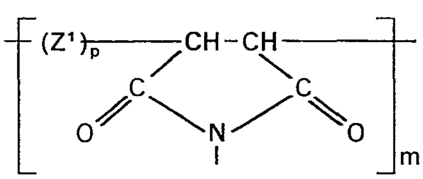 (VI)

bedeutet, worin p Null oder 1 und m die Zahlen 1 bis 250 bedeuten, $Z^1$ gegebenenfalls mit $C_1$ bis $C_{24}$-Alkyl oder Alkoxy, $C_2$ bis $C_{24}$-Alkenyl, Phenyl, $C_2$ bis $C_{24}$-Carbalkoxy, Carbophenoxy, $C_2$ bis $C_{24}$-Alkanoyloxy, Benzoyloxy, Halogen oder Cyano substituiertes Äthylen, $R^3$ H, gegebenenfalls Halogenatome substituiertes $C_1$ bis $C_{24}$-Alkyl, gegebenenfalls durch $-OH$, $-NH_2$ oder $C_1$ bis $C_{24}$-Alkyl substituiertes $C_6$ bis $C_{14}$-Aryl oder $C_7$ bis $C_{16}$-Aralkyl, $R^4$ Wasserstoff, $C_1$ bis $C_{24}$-Alkyl, eine Gruppe $-X-OH$ oder $-X-OCO-CH_2-CO-R$ und $R^5$ Wasserstoff, $-OH$, $-OR^3$, $-SR^3$, $-NHR^3$ oder $-X-OH$ sind, Q eine Gruppe $-Z-CO-$ ist, wobei die Carbonylgruppe an den Stickstoff der Formel II gebunden ist und Z Äthylen ist, das gegebenenfalls an ein 1,3-Homo- oder (Oxa-, Aza-)Heterodien addiert ist, welches seinerseits, gegebenenfalls partiell, Bestandteil eines Homo- oder (Oxa-, Thia-, Aza-)Heterocyclus mit 5 bis 40 Ringgliedern und 1 bis 10 Ringen sein kann, oder ferner Z Vinylen, gegebenenfalls durch eine Gruppe $-CON(R^6)R^7$, $-COOR^8$, $-COSR^8$ oder gegebenenfalls durch 4 Halogenatome substituiertes o-Phenylen oder die Gruppe $-CH_2-S-CH_2$ bedeutet, $R^6$ und $R^7$ gleich oder verschieden Wasserstoff oder $C_1$ bis $C_{12}$-Alkyl sind, und $R^8$ $C_1$ bis $C_{12}$-Alkyl oder eine Gruppe $-CH_2-COOR^9$, worin $R^9$ $C_1$ bis $C_{12}$-Alkyl ist, bedeutet oder Q eine ringschliessende Gruppe, die zu einem 5-10 gliedrigen Heterocyclus mit mindestens 2 Heteroatomen führt, und wenn n 2 ist, A eine der Gruppen der Formeln

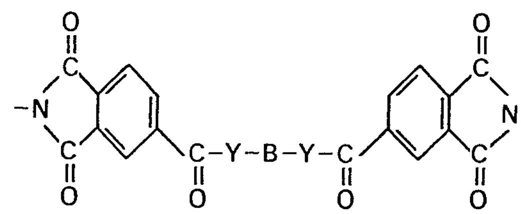 (VII)

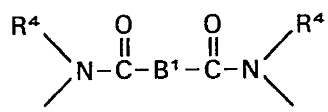 (VIII)

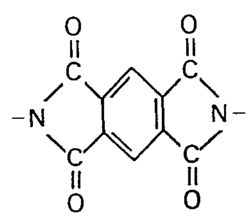 (IX)

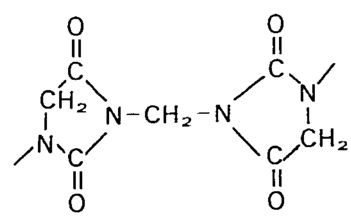 (X)

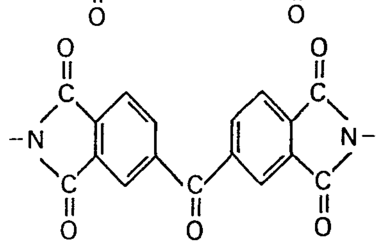 (XI)

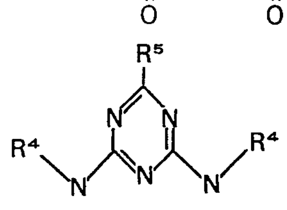

(XII)

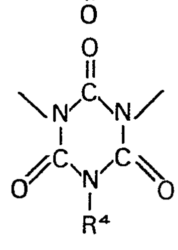 (XIII)

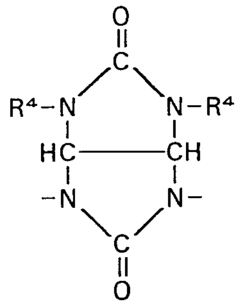

(XIV)

bedeutet, worin Y –O– oder –NH– sein kann, B $C_1$ bis $C_6$-Alkylen, p-Phenylen oder eine der Gruppen

$-[-(CH_2)_q O]_r-(CH_2)_q-$ oder
$-[-(CH_2)_q S]_r-(CH_2)_q-$

ist, worin q die Zahlen 1 bis 4 und r die Zahlen 1 bis 3 bedeuten, und $B^1$ eine direkte Bindung, B, $C_{10}$ bis $C_{14}$-Arylen oder $C_5$ bis $C_8$-Cycloalkylen bedeutet, und ferner
wenn n 3 ist, A eine Gruppe der Formel

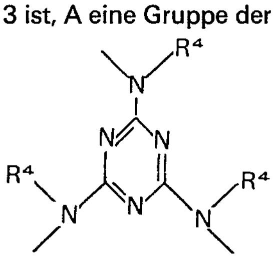

(XV)

ist, worin $R^4$ die oben angegebene Bedeutung hat.

Stellen etwaige Substituenten $C_1$ bis $C_{12}$-Alkyl dar, so handelt es sich um verzweigtes oder unverzweigtes Alkyl, wie z.B. Methyl, Äthyl, n-Propyl, Isopropyl, n-Butyl, tert.-Butyl, n-Pentyl, 2-Methylpentyl, Hexyl, 2,4-Dimethylpentyl, Octyl, 6-Methylheptyl, 2-Äthylhexyl, Decyl oder Dodecyl.

In der Bedeutung von $C_1$ bis $C_{24}$-Alkyl können etwaige Substituenten z.B. die oben angeführten Gruppen und dazu noch beispielsweise verzweigtes oder unverzweigtes Tridecyl, Tetradecyl, Hexadecyl, Octadecyl, Eicosyl, Docosyl, Tricosyl oder Tetracosyl darstellen.

Stellen etwaige Substituenten $C_1$ bis $C_6$-Alkylen dar, so handelt es sich um verzweigtes oder insbesondere geradkettiges Alkylen, wie beispielsweise Methylen, Äthylen, Tri-, Tetra-, Penta- oder Hexamethylen. Bevorzugt sind Methylen und insbesondere Äthylen und Trimethylen.

Stellen etwaige Substituenten $C_1$ bis $C_{24}$-Alkoxy dar, so handelt es sich z.B. um Methoxy, Äthoxy, n-Propoxy, Isopropoxy, n-Butoxy, tert.-Butoxy, n-Pentoxy, n-Hexyloxy, 2,4-Dimethylpentyloxy, n-Octyloxy, 2-Äthylhexyloxy, oder um verzweigtes oder unverzweigtes Decyloxy, Dodecyloxy, Tridecyloxy, Tetradecyloxy, Hexadecyloxy, Octadecyloxy, Eicosyloxy, Docosyloxy, Tricosyloxy oder Tetracosyloxy. Bevorzugt sind Methoxy und Äthoxy.

Stellen etwaige Substituenten $C_2$ bis $C_{24}$-Alkenyl dar, so handelt es sich beispielsweise um Vinyl, Allyl, Methallyl oder verzweigtes oder unverzweigtes 2-Butenyl, 2-Hexenyl, 3-Hexenyl, 2-Octenyl, 2-Dodecenyl, 3-Dodecenyl, 2-Tetradecenyl, 2-Hexadecenyl oder 2-Octadecenyl. Bevorzugt ist Allyl.

$C_2$ bis $C_{24}$-Carboalkoxy bedeutet insbesondere Carbomethoxy, Carboäthoxy, Carbopropoxy, Carbobutoxy und $C_2$ bis $C_{24}$-Alkanoyloxy ist bevorzugt Acetyloxy.

Stellen etwaige Substituenten Halogen dar, so handelt es sich vorzugsweise um Brom oder insbesondere um Chlor.

Bedeutet $B^1$ $C_{10}$ bis $C_{14}$-Arylen, so handelt es sich beispielsweise um 2,3- oder 1,8-Napthylen oder um 2,3-Anthracenylen.

Stellt $B^1$ $C_5$ bis $C_8$-Cycloalkylen, so handelt es sich insbesondere um Hexylen.

Bedeutet Z Äthylen, das gegebenenfalls an ein 1,3-Homo- oder (Oxa-, Aza-)Heterodien addiert ist, welches seinerseits, gegebenenfalls partiell, Bestandteil eines Homo- oder (Oxa-, Thia-, Aza-) Heterocyclus mit 5 bis 40 Ringgliedern und 1 bis 10 Ringen sein kann, so handelt es sich bei A beispielsweise um einen der Reste der Formeln

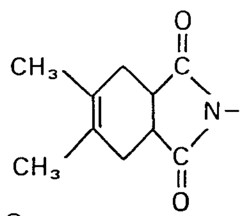

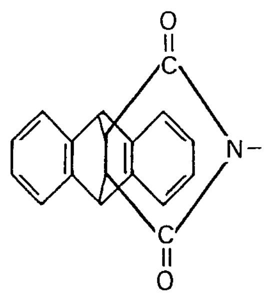

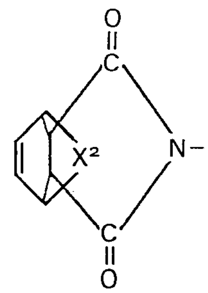

worin $X^2$ –O–, –S–, $-N(R^1)(R^2)-$ oder $-C(R^1)(R^2)-$ bedeutet.

Bedeutet Q eine ringschliessende Gruppe, die zu einem 5-10gliedrigen Heterocyclus mit mindestens 2 Heteroatomen führt, so kann es sich bei A beispielsweise um Reste der folgenden Formeln handeln

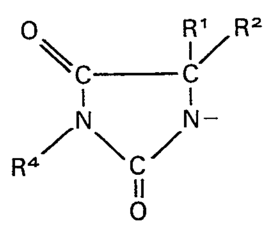

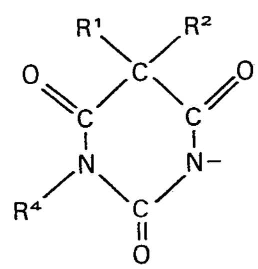

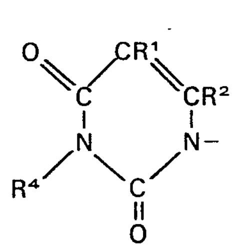

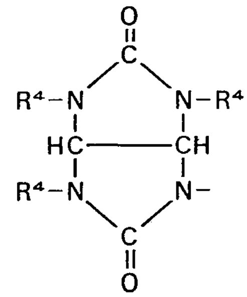

dementsprechend bedeutet Q beispielsweise jeweils eine Gruppe $-C(R^1)(R^2)-CO-N(R^4)-$, $-CO-C(R^1)(R^2)-CO-N(R^4)-$, $-CR^1=CR^2-CO-N(R^4)-$ oder

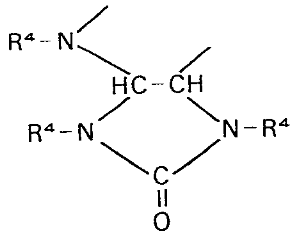

wobei der Stickstoff jeweils an die Carbonylgruppe der Formel II gebunden ist.

Bedeutet $R^3$ gegebenenfalls durch $-OH$, $-NH_2$ oder $C_1$ bis $C_4$-Alkyl substituiertes $C_6$ bis $C_{14}$-Aryl, so handelt es sich bevorzugt um gegebenenfalls 1,2- oder 3mal substituiertes Phenyl und insbesondere um Phenyl, 3,5-Dimethyl-4-hydroxyphenyl oder 3,5-Di-tert.-butyl-4-hydroxyphenyl.

Bedeutet $R^3$ gegebenenfalls durch $-OH$, $-NH_2$ oder $C_1$ bis $C_4$-Alkyl substituiertes $C_7$ bis $C_{16}$-Aralkyl, so handelt es sich bevorzugt um gegebenenfalls 1, 2- oder 3mal substituiertes Benzyl oder Phenäthyl und insbesondere um 3,-Dimethyl-4-hydroxybenzyl, 3,5-Di-tert.-butyl-4-hydroxybenzyl, 2-(3,5-Dimethyl-4-hydroxyphenyl)äthyl oder 2-(3,5-Di-tert.-butyl-4-hydroxyphenyl)-äthyl.

Besonders interessant sind Verbindungen der Formel I, worin n die Zahlen 1 oder 2, R Methyl oder Phenyl, $R^1$ und $R^2$ Wasserstoff, und X $C_2$ bis $C_3$-Alkylen oder eine Gruppe $-CH_2CH_2O-CH_2CH_2-$ bedeuten und,
wenn n 1 ist, A eine der Gruppen der Formeln II, III, IV oder V bedeutet, worin $R^3$ $C_1$ bis $C_{24}$-Alkyl, $R^4$ eine Gruppe $-X-OCO-CH_2-CO-R$, worin X und R die für diese Bevorzugung bereits angegebene Bedeutung haben, und $R^5$ $C_1$ bis $C_4$-Alkoxy sind, und,
wenn n 2 ist, A eine der Gruppen der Formeln VIII, IX, XI oder XIV bedeutet, worin $B^1$ eine p-Phenylengruppe ist, und $R^4$ Wasserstoff oder die Gruppe

$$-X-OCO-CH_2-CO-R$$

bedeutet, worin X und R die für diese Bevorzugung bereits angegebene Bedeutung haben.

Bevorzugt sind Verbindungen der Formel I, worin R Methyl und X $C_2$ bis $C_3$-Alkylen sind, n die Zahlen 1 oder 2 bedeuten und,
wenn n 1 ist, A eine Gruppe der Formel

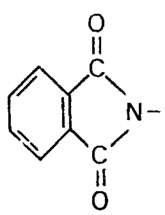

bedeutet und,
wenn n 2 ist, A eine der Gruppe der Formeln VIII, IX, XI oder XIV bedeutet, worin B' eine p-Phenylengruppe ist, und $R^4$ Wasserstoff oder die Gruppe $-X-OCO-CH_2-CO-R$ bedeutet, worin X und R die für diese Bevorzugung bereits angegebene Bedeutung haben.

Die Darstellung der erfindungsgemässen Verbindungen der Formel I erfolgt sehr glatt in Analogie zu allgemein bekannten Umesterungsreaktionen, durch Einwirkung eines β-Ketocarbonsäureesters der Formel XVI

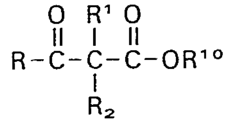 (XVI)

auf einem Alkanolamid der Formel XVII

$$A{-}(X{-}OH)_n \qquad (XVII)$$

worin n, A, R, $R^1$ und $R^2$ die oben angegebene Bedeutung haben, und $R^{10}$ Methyl oder Äthyl bedeutet.

Es ist vorteilhaft, diese Umesterung unter Zuhilfenahme einer Destillationskolonne vorzunehmen; der als Reaktionsprodukt gebildete Methyl- oder Äthylalkohol lässt sich damit am besten abtrennen. Die Temperatur für die Umesterung liegt im Bereich zwischen 50 und 200° C, vorzugsweise zwischen 120 und 160° C.

Wird totale Umesterung erstrebt, so ist es günstig, den Acetessigsäuremethylester in etwa der doppelten stöchiometrischen Menge einzusetzen. Unter stöchiometrischen Bedingungen wird nur eine partiell statistische Umsetzung erreicht. Diese Produkte sind aber auch wertvolle Stabilisatoren.

Die Alkanolamide, die als Ausgangsprodukte für die Herstellung der erfindungsgemässen Stabilisatoren dienen, sind an sich bekannt. Sollten einzelne von ihnen noch neu sein, so können sie in Analogie zu bekannten Reaktionen hergestellt werden, wie beispielsweise:

a) aus einem Carbonsäureanhydrid und einem Alkanolamin, z.B. nach der Gleichung

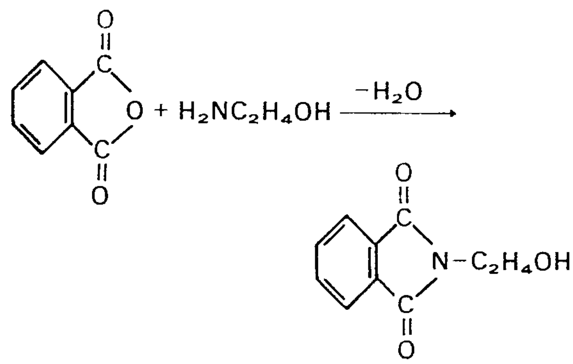


b) aus einem Carbonsäureester und Mono- oder Dialkanolamin, z.B. nach den Gleichungen

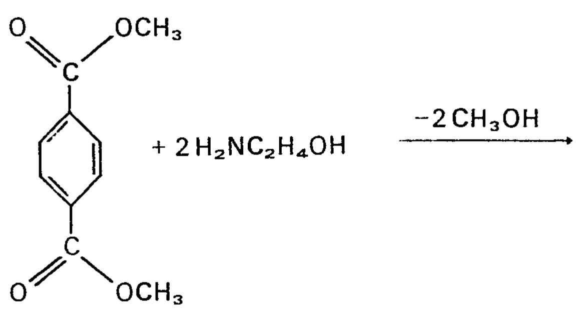

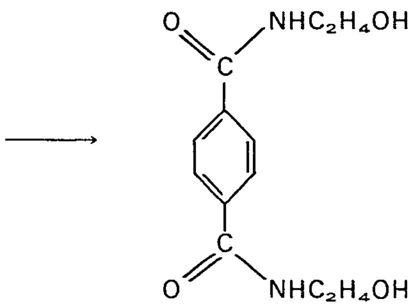

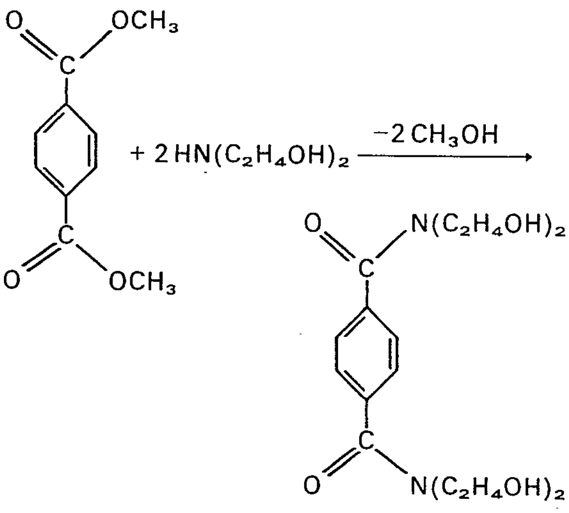


c) aus Cyanurchlorid und einem Mono- oder Dialkanolamin, z.B. nach den Gleichungen

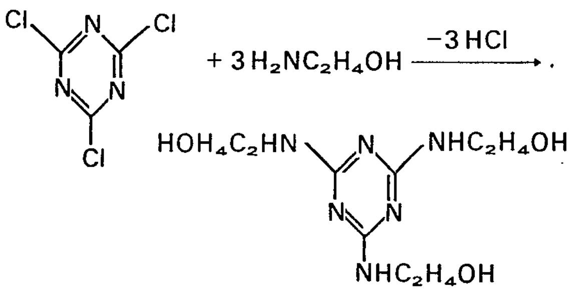


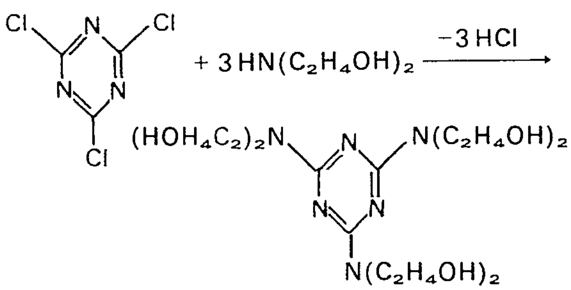


d) aus Cyanursäure und Äthylenoxyd, nach der Gleichung

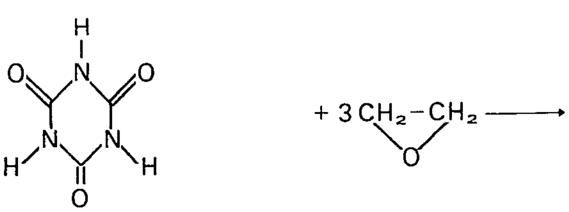

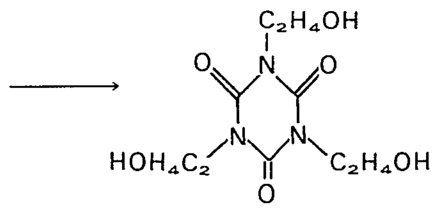

e) Aus einem Imid und Äthylenoxyd oder Formaldehyd, z.B. nach den Gleichungen

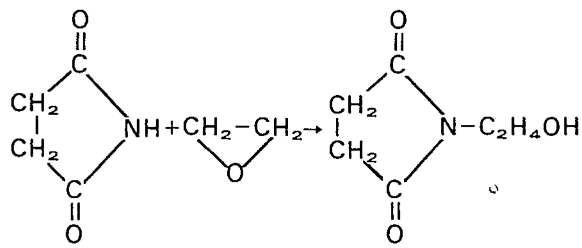

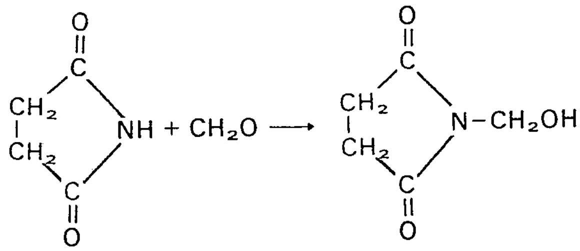

Bei den β-Ketocarbonsäureestern der Formel XVI handelt es sich um bekannte Verbindungen.

Die erfindungsgemässen Alkanolamid-β-ketocarbonsäureester eignen sich sehr gut zum Schutz gegen den Abbau durch Wärmeeinwirkung von chlorhaltigen Thermoplasten. Sie können einzeln oder untereinander vermischt den zu stabilisierenden Thermoplasten vor der Verarbeitung in üblichen Einrichtungen einverleibt werden, und zwar in jeweiligen Mengen von 0,05-5,0, bevorzugt 0,1-1,5 Gew.%, bezogen auf die gesamte Zusammensetzung.

Eine weitere Ausführungsform der Erfindung sind demnach stabilisierte Zusammensetzungen enthaltend ein chlorhaltiges thermoplastisches Polymer und als Stabilisator eine Verbindung oder ein Gemisch von Verbindungen der Formel I, bevorzugt zusammen mit anderen Stabilisatoren.

Als chlorhaltige Thermoplaste seien genannt: Polyvinylidenchlorid und bevorzugt Polymere aus oder auf der Grundlage von Vinylchlorid. Bevorzugt sind Suspensions- und Massepolymere und ausgewaschene, also emulgatorarme Emulsionspolymere. Beim Polyvinylchlorid kann es sich um weichmacherhaltiges oder um Hart-PVC handeln.

Als Comonomere für Thermoplaste auf der Grundlage von Vinylchlorid seien genannt: Vinylidenchlorid, Transdichloräthen, Äthylen, Propylen, Butylen, Maleinsäure, Acrylsäure, Fumarsäure oder Itaconsäure.

Vor, während oder nach der Zugabe des erfindungsgemässen Stabilisators oder Stabilisatorgemisches können je nach Verwendungszweck der Formmasse weitere Zusätze einverleibt werden.

Als weitere Additive, mit denen zusammen die erfindungsgemäss verwendbaren Stabilisatoren eingesetzt werden können, sind beispielsweise zu nennen:

Antioxydantien, wie 2,6-Dialkylphenole, Derivate von alkylierten Hydrochinonen, hydroxylierte Thiodiphenyläther, Alkylidenbisphenole, O-, N- und S-Benzylverbindungen, hydroxybenzylierte Malonester, Hydroxybenzylaromaten, 2-Triazinverbindungen, Amide der β-(3,5-Ditert.-butyl-4-hydroxyphenyl)propionsäure, Ester der β-(3,5-Ditert.-butyl-4-hydroxyphenyl)propionsäure, Ester der β-(5-tert.-Butyl-4-hydroxy-3-methylphenyl)propionsäure, Ester der 3,5-Ditert.-butyl-4-hydroxyphenylessigsäure, Acylaminophenole, Benzylphosphonate, Aminoarylderivate, UV-Absorber und Lichtschutzmittel, wie 2-(2'-Hydroxyphenyl)benztriazole, 2,4-Bis-(2'-hydroxyphenyl)-6-alkyl-s-triazine, 2-Hydroxybenzophenone, 1,3-Bis-(2'-hydroxybenzoyl)benzole, Ester von gegegenenfalls substituierten Benzoesäuren, Acrylate, des weiteren Nickelverbindungen, sterisch gehinderte Amine, Oxalsäurediamide, Metalldesaktivatoren, Epoxide, Phosphite, peroxidzerstörende Verbindungen, Polyamidstabilisatoren, basische Costabilisatoren, Nukleierungsmittel oder sonstige Zusätze, wie z.B. Weichmacher, Gleitmittel, Emulgatoren, Füllstoffe, Russ, Asbest, Kaolin, Talk, Glasfasern, Pigmente, optische Aufheller, Flammschutzmittel, Antistatica.

Beispiele für weitere Additive, mit denen zusammen die erfindungsgemäss verwendbaren Stabilisatoren eingesetzt werden können, finden sich in der DE-OS Nr. 2427853 auf Seiten 18 bis 24.

Bevorzugte Costabilisatoren sind Ca/Zn-Carboxylate und Phosphite, wie z.B. Trialkyl-, Triaryl-, Trialkaryl- oder gemischte Alkyl/Aryl-Phosphite.

Die nachfolgenden Beispiele dienen der näheren Erläuterung der Erfindung. Teile sind dabei Gewichtsteile und Prozente Gewichtsprozente.

*Herstellungsbeispiele*

*Beispiel 1:*

46,5 g Adipinsäurebisäthanolamid (0,2 mol) werden unter Umesterungsbedingungen bei 150°C während 3 h unter Rühren mit 93 g Acetessigsäuremethylester (0,8 mol) erhitzt. Nach dem Abdestillieren des entstandenen Methanols wird unter Vakuum der überschüssige Acetessigester entfernt. Der Rückstand wird aus Aceton unter Aktivkohlezusatz umkristallisiert. Man erhält 58,1 g (73% d.Th.) *Adipinsäurebis-2-äthanolamidacetoacetat* mit Smp. 98-100° C.

$O=C-NHC_2H_4OCOCH_2COCH_3$
$|$
$(CH_2)_4$
$|$
$O=C-NHC_2H_4OCOCH_2COCH_3$

*(Stabilisator 1)*

*Analyse:*

| | | | |
|---|---|---|---|
| Berechnet: | C 54,0 | H 7,1 | N 7,0% |
| Gefunden: | C 53,5 | H 7,1 | N 7,3% |

*Beispiel 2:*

Ersetzt man in Beispiel 1 das Adipinsäurebisäthanolamid durch die äquivalente Menge Bernsteinsäurebisäthanolamid, so erhält man nach Umkristallisation 51,0 g (69% d.Th.) *Bernsteinsäurebis-2-äthanolamidacetoacetat* mit Smp. 108-110° C.

$O=C-NHC_2H_4OCOCH_2COCH_3$
$|$
$(CH_2)_2$
$|$
$O=C-NHC_2H_4OCOCH_2COCH_3$

*(Stabilisator 2)*

*Analyse:*

| | | | |
|---|---|---|---|
| Berechnet: | C 51,6 | H 6,5 | N 7,5% |
| Gefunden: | C 51,0 | H 6,5 | N 7,8% |

*Beispiel 3:*

Ersetzt man in Beispiel 1 das Adipinsäurebisäthanolamid durch die äquivalente Menge Oxalsäurebisäthanolamid, so erhält man nach Umkristallisation 45,2 g (68% d. Th.) *Oxalsäurebis-2-äthanolamidacetoacetat* mit Smp. 114-116° C.

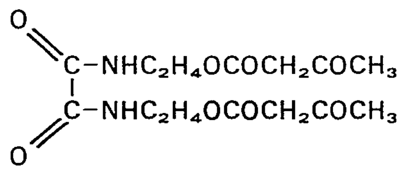

*(Stabilisator 3)*

*Analyse:*

| | | | |
|---|---|---|---|
| Berechnet: | C 48,8 | H 5,9 | N 8,1% |
| Gefunden: | C 48,8 | H 5,8 | N 8,2% |

*Beispiel 4:*

Ersetzt man in Beispiel 1 das Adipinsäurebisäthanolamid durch die äquivalente Menge Terephthalsäurebisäthanolamid, so erhält man 69,6 g (83% d. Th.) *Terephthalsäurebis-2-äthanolamidacetoacetat* mit Smp. 145° C.

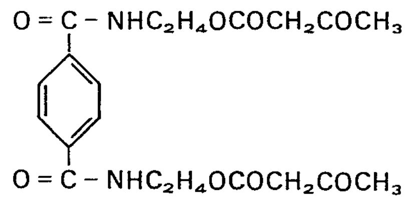

*Stabilisator 4)*

*Analyse:*

| | | | |
|---|---|---|---|
| Berechnet: | C 57,14 | H 5,71 | N 6,66% |
| Gefunden: | C 59,20 | H 6,60 | N 6,40% |

Die Bisäthanolamide, die in den oben beschriebenen Beispielen als Ausgangsprodukte dienen können nach bekannten Methoden aus den entsprechenden Carbonsäurediäthylestern durch Umsetzung mit Äthanolamin hergestellt werden, wie beispielsweise hier für das Bernsteinsäurebisäthanolamid beschrieben ist: 62,2 g Bernsteinsäurediäthylester (0,357 mol) werden unter Rühren während 3 h bei 130-140° C mit 48 g Äthanolamin (0,785 mol) erhitzt. Nachdem das entstandene Methanol abdestilliert wurde, wird der bei 110° C erstarrende Rückstand in Aceton digeriert, filtriert und abgesaugt. Das Rohprodukt wird aus absolutem Äthanol umkristallisiert. Man erhält 69 g (95% d. Th.) eines farblosen Pulvers mit Smp. 155-157° C.

*Analyse:*

| | | | |
|---|---|---|---|
| Berechnet: | C 47,0 | H 7,9 | N 13,7% |
| Gefunden: | C 46,7 | H 7,7 | N 13,7% |

*Beispiel 5:*

148,1 g Phthalsäureanhydrid (1 mol) werden unter Rühren in 67,2 g (1,1 mol) Äthanolamin gegeben (exotherm, Innentemperatur IT~100° C). Es wird 2 h bei 140° C Badtemperatur (IT~120° C) gerührt und dann in 30 min unter Vakuum Wasser und überschüssiges Äthanolamin abgezogen. Es werden nun 500 g Acetessigsäuremethylester (4,2 mol) zugegeben und unter schwachem Vakuum (120-150 Torr) während 2 h bei 120° C Badtemperatur das entstehende Methanol abdestilliert. Danach wird der überschüssige Acetessigester unter Vakuum bei 120° C Badtemperatur innert 2 h abgezogen. Der Rückstand wird in wenig kaltem Methanol umkristallisiert, dann abfiltriert und getrocknet. Man erhält 209 g (76% d.Th.) eines schwachgelben Pulvers von *1-(Acetoacetyloxy-2-äthyl)phthalimid* mit Smp. 83-87° C.

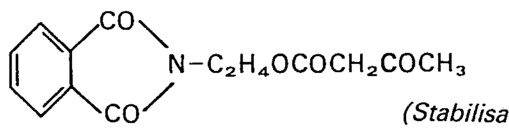

*(Stabilisator 5)*

*Beispiel 6:*

In eine Lösung von 122 g Äthanolamin (2 mol) in 300 ml Dimethylformamid werden in kleine Portionen unter Rühren 218 g Pyromellitsäuredianhydrid (1 mol) eingetragen (exotherm; Schwarzfärbung). Anschliessend wird am Rückfluss erhitzt (142° C), ca. 80 ml Dimethylformamid/Wasser-Gemisch abdestilliert, bis zur konstanten Siedetemperatur des Dimethylformamid (153° C). Nach dem Abkühlen wird der kristalline Niederschlag abfiltriert und mit 300 ml Acetessigsäuremethylester am Umesterungskühler so lange unter Rückfluss gekocht, bis das entstandene Methanol abdestilliert ist. Überschüssiger Acetessigester wird abgezogen. Mehrmaliges Umkristallisieren aus Aceton liefert 384 g (80% d.Th.) reines *Bis-N-(acetoacetyloxy-2-äthyl)pyromellitsäurediimid* mit Smp. 161-163° C.

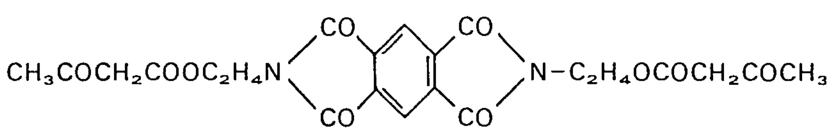

*(Stabilisator 6)*

*Analyse:*

| | | | |
|---|---|---|---|
| Berechnet: | C 55,93 | H 4,27 | N 5,93% |
| Gefunden: | C 56,02 | H 4,33 | N 6,00% |

*Beispiel 7:*

Ersetzt man in Beispiel 6 das Äthanolamin durch die äquivalente Menge 3-Amino-1-propanol, so erhält man als Endprodukt 429,0 g (86% d.Th.) reines *Bis-N-(acetoacetyloxy-3-propyl)-pyromellitsäurediimid* mit Smp. 115-117° C.

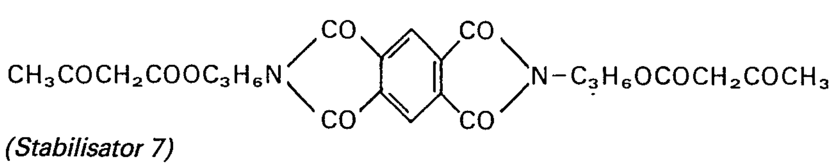

*(Stabilisator 7)*

*Analyse:*

| | | | |
|---|---|---|---|
| Berechnet: | C 57,54 | H 4,79 | N 5,59% |
| Gefunden: | C 57,73 | H 4,81 | N 5,63% |

*Beispiel 8:*

Ersetzt man im Beispiel 1 das Adipinsäurebisäthanolamid durch die äquivalente Menge Trishydroxyäthylisocyanurat, so erhält man nach abziehen der flüchtigen Bestandteile im Vakuum 60,0 g (86% d.Th.) *Isocyanursäuretris-N-(2-äthylacetoacetat)* als viskose Substanz mit einem Brechungsindex $n_D^{20}$ 1,4991.

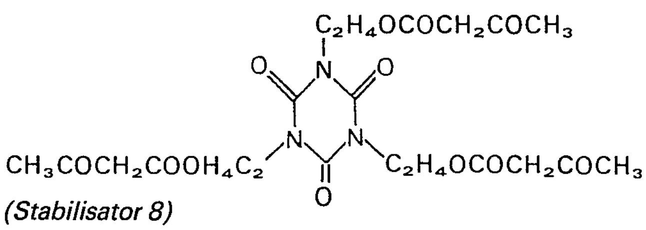

*(Stabilisator 8)*

Analog ist auch 1,1-Methylen-bis-[3-(hydroxyäthyl)-5,5-dimethylhydantoin] umsetzbar.

*Beispiel 9:*

a) In eine Lösung von 98 g Maleinsäureanhydrid (1 mol) in 200 ml Xylol werden portionenweise 178 g Anthracen (1 mol) unter Rühren zugegeben. Dann wird ca. 2 h am Rückfluss gekocht und nach dem Abkühlen der Rückstand abgesaugt. Man erhält 257 g Anhydrid der Formel

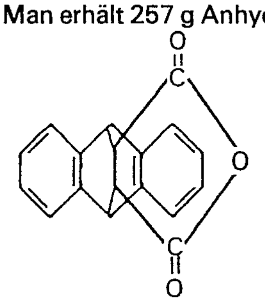

in Form eines weissen Pulvers mit Smp. 263-267° C.

b) In eine Lösung von 41,1 g des wie unter a beschrieben erhaltenen Anhydrids (0,15 mol) in 100 ml Chloroform wird unter Rühren eine Lösung von 9,2 Äthanolamin (0,15 mol) in 20 ml Chloroform zugetropft. Es entsteht bei exothermer Reaktion ein Niederschlag. Man gibt überschüssiges Toluol zu und kocht 5 h am Umesterungskühler bis das entstandene Wasser abgezogen ist. Der Rückstand wird abgesaugt, mit Chloroform gewaschen und getrocknet. Man erhält 46,4 g Äthanolimid der Formel

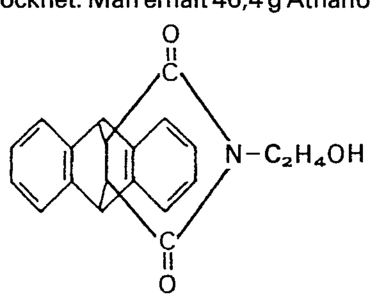

in Form eines farblosen Pulvers mit Smp. 216-218° C.

c) 31,7 g des wie unter b beschrieben erhaltenen Äthanolimids (0,1 mol), 23,2 g Acetessigsäuremethylester (0,2 mol) und 0,5 ml Tetrabutyltitanat werden zusammen unter Rühren auf 160° C während 15 h am Umesterungskühler erhitzt, wobei das gebildete Methanol abgezogen wird. Der dunkle Rückstand wird in Aceton gelöst, mit Aktivkohle behandelt und abfiltriert. Die Lösung wird eingeengt bis ein gelber klebriger Rückstand zurückbleibt. Dieser wird in Diisopropyläther digeriert, abgesaugt und getrocknet. Man erhält 30 g (75% d.Th.) des Äthanolimidacetoacetats der Formel

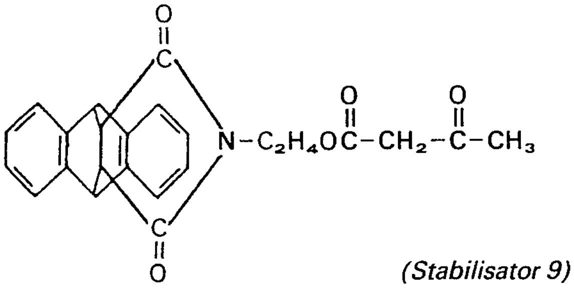

*(Stabilisator 9)*

in Form von Farblosen Kristallen mit Smp. 128-130° C.

*Beispiel 10:*

a) 38,4 g (0,2 mol) Trimellitsäureanhydrid werden in 200 ml Toluol vorgelegt. Unter Rühren tropft man 12,3 g (0,2 mol) Äthanolamin zu, wobei unter Eiskühlung die Temperatur auf maximal 45° C gehalten wird. Nach beendeter Zugabe wird unter Rückfluss erhitzt und das Reaktionswasser azeotrop ausgekreist. Nach Abziehen der flüchtigen Bestandteile wird in wenig Äthanol aufgenommen und gekühlt. Der Niederschlag wird abgesaugt und getrocknet, wobei 53 g (82% d.Th.) einer farblosen Substanz mit Smp. 197-198° C erhalten wird. Es handelt sich um 1-(2-Hydroxyäthyl)-4-hydroxycarbonylphthalimid.

b) 35,3 g (0,15 mol) 1-(2-Hydroxyäthyl)-4-hydroxycarbonylphthalimid aus a werden in 250 ml Methanol suspendiert. Man leitet dann unter Erhitzen am Rückfluss HCl-Gas bis zur Sättigung ein. Die klare Lösung wird etwas eingeengt und gekühlt, wobei ein Produkt ausfällt, das nach dem Trocknen 35,2 g (94% d.Th.) einen Schmelzpunkt von 81-82° C zeigt. Es handelt sich um 1-(2-Hydroxyäthyl)-4-methoxycarbonylphthalimid.

Durch Umesterung mit Stearylalkohol ist das entsprechende Stearylprodukt in quantitativer Ausbeute erhältlich. Durch Umesterung mit Äthylacetoacetat ist der entsprechende Acetessigester zugänglich.

c) 24,9 g (0,1 mol) 1-(2-Hydroxyäthyl)-4-methoxycarbonylphthalimid aus b werden in 100 ml Methylacetoacetat gelöst. Nach zweistündigem Erhitzen auf 145° C ist die Umesterung beendet. Nach Abziehen der flüchtigen Bestandteile im Vakuum erhält man 30 g (90% d.Th.) eines nahezu farblosen hochviskosen Öles. Es handelt sich um *1-(Acetoacetyloxy-2-äthyl)methoxycarbonylphthalimid*

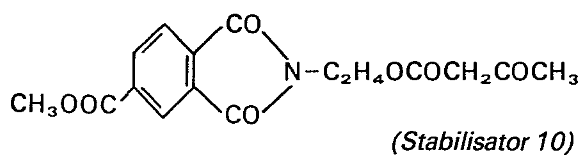

*(Stabilisator 10)*

Durch Umesterung mit Stearylalkohol ist daraus ein langkettig modifiziertes wachsartiges Acetoacetat erhältlich.

*Beispiel 11:*

a) 1620 g (12 mol) Diäthyloxalat werden langsam in 300 ml Äthanol mit 183 g (3 mol) Äthanolamin versetzt, bei einer maximalen Temperatur von 0° C. Dann werden die flüchtigen Bestandteile im Vakuum abdestilliert. Der Rückstand wird im Hochvakuum (Sdp. 160° C/0,001 Torr) destilliert und man erhält 266 g (55% d.Th.) Oxalsäureäthylester-(2-äthanolamid) als viskoses Öl.

Durch Umesterung mit Stearylalkohol ist das entsprechende wachsartige Stearylderivat zugänglich, aus welchem durch Umesterung mit Äthylacetoacetat die entsprechende Acetessigesterverbindung erhältlich ist.

b) 80,6 g (0,5 mol) Oxalsäureäthylester-(2-äthanolamid) aus a werden bei 160° C in 200 ml Äthylacetoacetat umgeestert. Nach Abziehen der flüchtigen Bestandteile verbleibt ein hochviskoses Öl, das alsbald erhärtet. Nach Digerieren in Diäthyläther erhält man 85 g (70% d.Th.) *Oxalsäureäthylester-(2-äthanolamidacetoacetat)* als hellgelbes Pulver mit Smp. 39-40° C

$C_2H_5OCO-CONHC_2H_4OCOCH_2COCH_3$

*(Stabilisator 11)*

Durch Umesterung mit Stearylalkohol lässt sich ein langkettig modifiziertes wachsartiges Produkt erhalten.

*Beispiel 12:*

a) 146,2 g (0,5 mol) β-(3,5-Ditert.-butyl-4-hydroxy)propionsäuremethylester werden mit 30,5 g (0,5 mol) Äthanolamin bei 160° C umgesetzt. Nach Abziehen der flüchtigen Bestandteile erhält man in quantitativer Ausbeute ein Öl, das alsbald perlmutterartig erstarrt. Nach dem Umkristallisieren aus Toluol erhält man 148 g (92% d.Th.) β-(3,5-Ditert.-butyl-4-hydroxy)propionsäure-(2-äthanolamid) mit Smp. 122-123° C.

b) 32,0 g (0,1 mol) β-(3,5-Ditert.-butyl-4-hydroxy)propionsäure-(2-äthanolamid) aus a werden in 150 ml Methylacetoacetat bei 145° C umgeestert. Nach Abziehen der flüchtigen Bestandteile verbleiben 40 g (98% d.Th.) β-*(3,5-Ditert.-butyl-4-hydroxy)propionsäure-(2-äthanolamidacetoacetat)* als hochviskoses, nahezu farbloses Öl

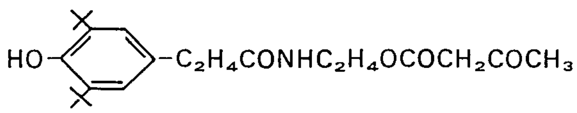

+ = tert.-Butyl *(Stabilisator 12)*

In Analogie zu den in den Beispielen 1 bis 12 beschriebenen Verfahren können aus entsprechenden bekannten Ausgangsstoffen folgende weitere erfindungsgemässe Stabilisatoren erhalten werden:

Ameisensäure-(2-äthanolamidacetoacetat)
$HCONHC_2H_4OCOCH_2COCH_3$

Trichloressigsäure-(2-äthanolamidacetoacetat)
$Cl_3CCONHC_2H_4OCOCH_2COCH_3$

p-Aminobenzoesäure-(2-äthanolamidacetoacetat)

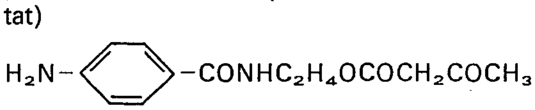

p-Hydroxybenzoesäure-(2-äthanolamidacetoacetat)

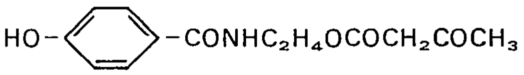

3,5-Ditert.-butyl-4-hydroxybenzoesäure-(2-äthanolamidacetoacetat)

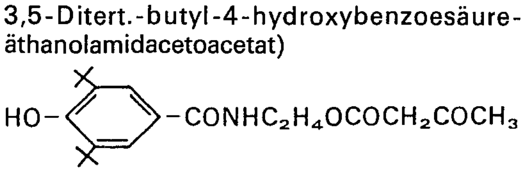

3-Thia-4-(3,5-ditert.-butyl-4-hydroxy)buttersäure-(2-äthanolamidacetoacetat)

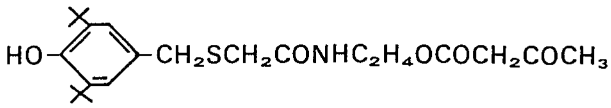

1-(Acetoxyacetyloxy-2-äthyl)-3,4,5,6-tetrachlorphthalimid

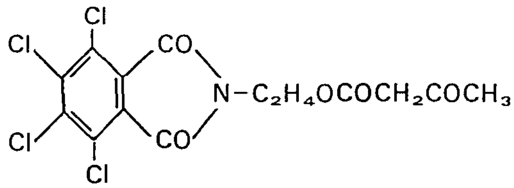

1-(Acetoacetyloxy-2-äthyl)-3,4,5,6-tetrabromphthalimid

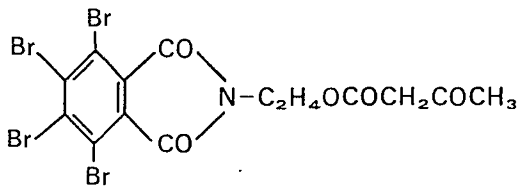

sowie Verbindungen der Formeln:

$S(CH_2-CO-NHC_2H_4OCOCH_2COCH_3)_2$
$S(C_2H_4-CO-NHC_2H_4OCOCH_2COCH_3)_2$
$S-CH_2-CO-NHC_2H_4OCOCH_2COCH_3$
$S-CH_2-CO-NHC_2H_4OCOCH_2COCH_3$
$S-C_2H_4-CO-NHC_2H_4OCOCH_2COCH_3$
$S-C_2H_4-CO-NHC_2H_4OCOCH_2COCH_3$
$(C_2H_5O)_2POCH_2-CO-NHC_2H_4OCOCH_2COCH_3$
$(C_2H_5O)_2POC_2H_4-CO-NHC_2H_4OCOCH_2COCH_3$
$C_{18}H_{37}OCOCH=CHCO-NHC_2H_4OCOCH_2COCH_3$

*Applikationsbeispiele*

Eine Trockenmischung bestehend aus 100 Teilen S-PVC (K-Wert 58), 2 Teilen epoxidiertem Sojabohnenöl, 0,5 Teilen Trisnonylphenylphosphit, 0,6 Teilen Ca-Stearat, 0,4 Teilen Zn-Stearat und der in der nachstehenden Tabelle angegebenen Menge an erfindungsgemässen Stabilisator wird auf einem Mischwalzwerk 5 min bei 180° C gewalzt. Vom gebildeten Walzfell werden Testfolienstücke von 0,3 mm Dicke entnommen.

Die Folienproben werden in einem Ofen bei 180° C thermisch belastet und im zeitlichen Abstand von 15 min an einer Probe der Yellowness-Index (YI) nach ASTMD 1925-70 bestimmt.

Die Ergebnisse sind in der nachstehenden Tabelle zusammengefasst.

| Test Nr. | Stabilisator | Teile | Walzfolie | Yellowness-Index nach | |
|---|---|---|---|---|---|
| | | | | 15 min | 30 min |
| 1 | — | — | 45,2 | 75,7 | 92,2 |
| 2 | Stabilisator 5 | 0,8 | 11,2 | 20,1 | 51,7 |
| 3 | Stabilisator 7 | 0,8 | 9,0 | 17,3 | 49,0 |
| 4 | Stabilisator 8 | 0,8 | 7,2 | 10,8 | 18,9 |

Dieser Versuch wurde in gleicher Weise wiederholt mit der Ausnahme, dass 4 Teile epoxidiertes Sojabohnenöl, 0,4 Teile Ca-Stearat, 0,2 Zn-Stearat und anstatt Trisnonylphenylphosphit die in der nachstehenden Tabelle angegebene Menge Phosphit A (Gemisch aus $C_6H_5O\text{-}P(OC_{14}H_{29})_2$ und $C_{14}H_{29}O\text{-}P(OC_6H_5)_2$ eingesetzt wurden.

Die Ergebnisse sind in der nachstehenden Tabelle zusammengefasst:

| Test Nr. | Stabilisator | Teile | Walzfolie | Yellowness-Index nach | |
|---|---|---|---|---|---|
| | | | | 15 min | 30 min |
| 5 | Stabilisator 5<br>Phosphit A | 0,6<br>0,3 | 6,7 | 12,6 | 33,0 |
| 6 | Stabilisotor 5<br>Phosphit A | 0,6<br>0,6 | 6,0 | 13,0 | 21,5 |

## Patentansprüche

1. Verbindungen der Formel I

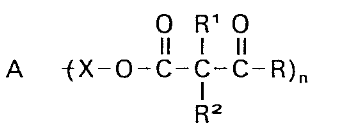 (I)

worin n die Zahlen 1 bis 3, R $C_1$ bis $C_{24}$-Alkyl oder Phenyl, $R^1$ und $R^2$ unabhängig voneinander Wasserstoff oder R und X gegebenenfalls durch $-O-$ unterbrochenes $C_1$ bis $C_6$-Alkylen bedeuten und, wenn n 1 ist, A eine der Gruppen der Formeln

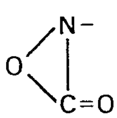 (II)

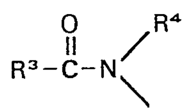 (III)

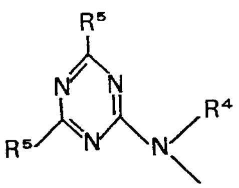 (IV)

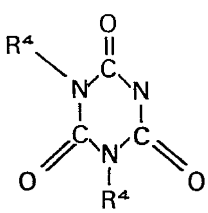 (V)

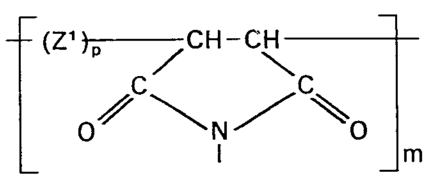 (VI)

bedeutet, worin p Null oder 1 und m die Zahlen 1 bis 250 bedeuten, $Z^1$ gegebenenfalls mit $C_1$ bis $C_{24}$-Alkyl oder Alkoxy, $C_2$ bis $C_{24}$-Alkenyl, Phenyl, $C_2$ bis $C_{24}$-Carbalkoxy, Carbophenoxy, $C_2$ bis $C_{24}$-Alkanoyloxy, Benzoyloxy, Halogen oder Cyano substituiertes Äthylen, $R^3$ H, gegebenenfalls durch Halogenatome substituiertes $C_1$ bis $C_{24}$-Alkyl, gegebenenfalls durch $-OH-$ $-NH_2$ oder $C_1$ bis $C_4$-Alkyl substituiertes $C_6$ bis $C_{14}$-Aryl oder $C_7$ bis $C_{16}$-Aralkyl, $R^4$ Wasserstoff, $C_1$ bis $C_{24}$-Alkyl, eine Gruppe $-X-OH$ oder $-X-OCO-CH_2-CO-R$ und $R^5$ Wasserstoff, $-OH$, $-OR^3$, $-SR^3$, $-R^3$, $-NHR^3$ oder $-X-OH$ sind, Q eine Gruppe $-Z-CO$ ist, wobei die Carbonylgruppe an den Stickstoff der Formel II gebunden ist und Z Äthylen ist, das gegebenenfalls an ein 1,3-Homo- oder (Oxa-, Aza-)Heterodien addiert ist, welches seinerseits, gegebenenfalls partiell, Bestandteil eines Homo- oder (Oxa-, Thia-, Aza-)Heterocyclus mit 5 bis 40 Ringgliedern und 1 bis 10 Ringen sein kann oder ferner Z Vinylen, gegebenenfalls durch eine Gruppe $-CON(R^6)R^7$, $-COOR^8$, $-COSR^8$ oder gegebenenfalls durch 4 Halogenatome substituiertes o-Phenylen oder die Gruppe $-CH_2-S-CH_2-$ bedeutet, $R^6$ und $R^7$ gleich oder verschieden Wasserstoff oder $C_1$ bis $C_{12}$ alkyl sind und $R^8$ $C_1$ bis $C_{12}$-Alkyl oder eine Gruppe $-CH_2-COOR^9$, worin $R^9$ $C_1$ bis $C_{12}$-Alkyl ist, bedeutet oder Q eine ringschliessende Gruppe, die zu einem 5-10gliedrigen Heterocyclus mit mindestens 2 Heteroatomen führt, und wenn n 2 ist, A eine der Gruppen der Formeln

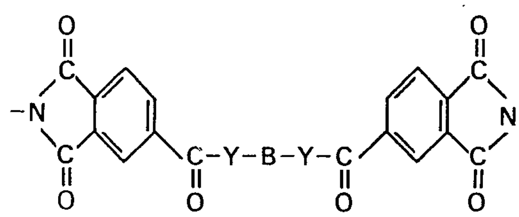 (VII)

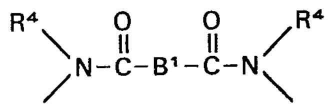 (VIII)

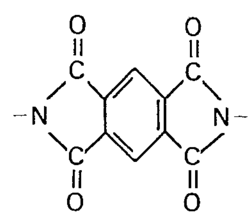 (IX)

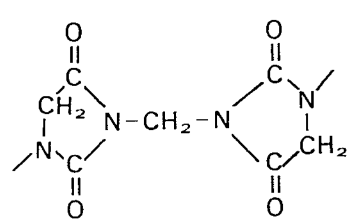 (X)

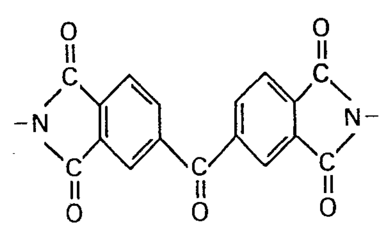 (XI)

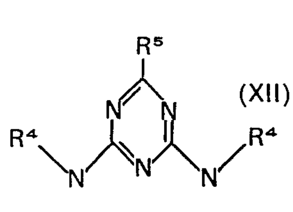 (XII)

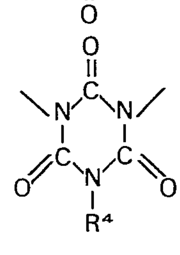 (XIII)

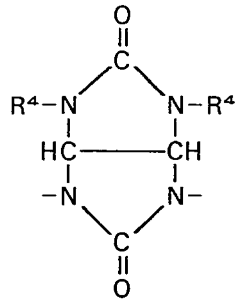

(XIV)

bedeutet, worin Y $-O-$ oder $-NH-$ sein kann, B $C_1$ bis $C_6$-Alkylen, p-Phenylen oder eine der Gruppen $[-(CH_2)_q-O]_r-(CH_0)-_q$ oder $[-(CH_2)_q-S-]_r-(CH_2)-_q$ ist, worin q die Zahlen 1 bis 4 und r die Zahlen 1 bis 3 bedeuten, und $B^1$ eine direkte Bindung, B, $C_{10}$ bis $C_{14}$-Arylen oder $C_5$ bis $C_8$-Cycloalkylen bedeutet, und ferner,
wenn n 3 ist, A eine Gruppe der Formel

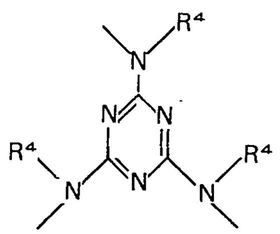

(XV)

ist, worin $R^4$ die oben angegebene Bedeutung hat.

2. Verbindungen gemäss Anspruch 1 der Formel (I), worin A eine Gruppe der Formel (II) ist und Q eine der Gruppen $-C(R^1)(R^2)-CO-N-(R^4)-$, $-CO-C(R^1)(R^2)-CO—N(R^4)-$, $-CR^1=CR^2-CO-N(R^4)-$ oder

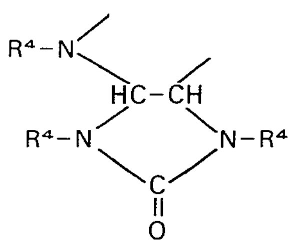

bedeutet, wobei der Stickstoff jeweils an die Carbonylgruppe der Formel (II) gebunden ist, und $R^4$ die in Anspruch 1 angegebene Bedeutung hat.

3. Verbindungen gemäss Anspruch 1 der Formel (I), worin n die Zahlen 1 oder 2, R Methyl oder Phenyl, $R^1$ und $R^2$ Wasserstoff, und X $C_2$ bis $C_3$-Alkylen oder eine Gruppe $-CH_2CH_2-O-CH_2CH_2-$ bedeuten und,
wenn n 1 ist, A eine der Gruppen der Formeln (II), (III), (IV) oder (V) bedeutet, worin $R_3$ $C_1$ bis $C_{24}$-Alkyl, $R_4$ eine Gruppe $-X-OCO-CH_2-CO-R$, worin X und R die in diesem Anspruch bereits angegebene Bedeutung haben, und $R^5$ $C_1$ bis $C_6$-Alkoxy sind, und,
wenn n 2 ist, A eine der Gruppen der Formeln (VIII), (IX), (XI) oder (XIV) bedeutet, worin $B^1$ eine p-Phenylengruppe ist und $R^4$ Wasserstoff oder die Gruppe $-X-OCO-CH_2-CO-R$ bedeutet, worin X und R die in diesem Anspruch bereits angegebene Bedeutung haben.

4. Verbindungen gemäss Anspruch 1 der Formel (I), worin R Methyl und X $C_2$ bis $C_3$-Alkylen sind, n die Zahlen 1 oder 2 bedeutet und, wenn n 1 ist, A eine Gruppe der Formel

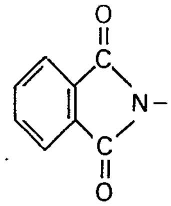

bedeutet und,
wenn n 2 ist, A eine der Gruppen der Formeln (VIII), (IX), (XI) oder (XIV) bedeutet, worin B' eine p-Phenylengruppe ist und $R^4$ Wasserstoff oder die Gruppe $-X-OCO-CH_2-CO-R$ bedeutet, worin X und R die für diese Bevorzugen bereits angegebene Bedeutung haben.

5. Stabilisierte Zusammensetzung enthaltend ein chlorhaltiges thermoplastisches Polymer und als Stabilisator eine Verbindung oder ein Gemisch von Verbindungen gemäss Anspruch 1 in einer Menge von 0,05 bis 5,0 Gew.%, bezogen auf die gesamte Zusammensetzung.

6. Stabilisierte Zusammensetzung gemäss Anspruch 5, dadurch gekennzeichnet, dass sie zusätzlich noch andere Stabilisatoren enthält.

7. Stabilisierte Zusammensetzung gemäss Anspruch 6, dadurch gekennzeichnet, dass einer der zusätzlichen Stabilisatoren Ca/Zn-Carboxylat ist.

8. Stabilisierte Zusammensetzung gemäss Anspruch 6. dadurch gekennzeichnet, dass einer der zusätzlichen Stabilisatoren ein Phosphit ist.

9. Stabilisierte Zusammensetzung gemäss Anspruch 5, dadurch gekennzeichnet, dass der chlorhaltige Thermoplast ein Polymer aus oder auf der Grundlage von Vinylchlorid ist.

10. Verwendung einer Verbindung gemäss Anspruch 1 zum Stabilisieren von chlorhaltigen thermoplastischen Polymeren.

**Claims**

1. A compound of the formula I:

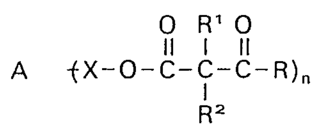

(I)

wherein n is an integer from 1 to 3, R is $C_1$-$C_{24}$-alkyl or -phenyl, each of $R^1$ and $R^2$ independently is hydrogen or R and X are $C_1$-$C_6$-alkylene which can be interrupted by $-O-$, and, if n is 1, A is one of the groups of the formulae:

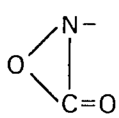

(II)

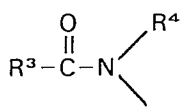

(III)

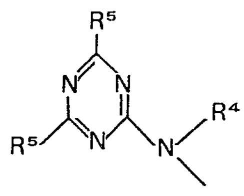

(IV)

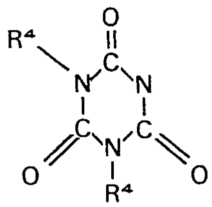

(V)

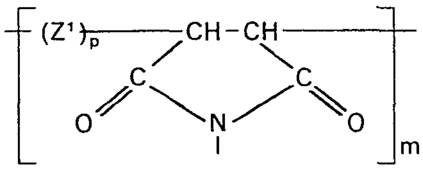

(VI)

wherein p is 0 or 1 and m is an integer from 1 to 250, $Z^1$ is ethylene which can be substituted by $C_1$-$C_{24}$-alkyl or $C_1$-$C_{24}$-alkoxy, $C_2$-$C_{24}$-alkenyl, phenyl, $C_2$-$C_{24}$-carbalkoxy, carbophenoxy, $C_2$-$C_{24}$-alkanoyloxy, benzoyloxy, halogen or cyano, $R^3$ is hydrogen, $C_1$-$C_{24}$-alkyl which can be substituted by halogen atoms, $C_6$-$C_{14}$-aryl or $C_7$-$C_{16}$-aralkyl, each of which can be substituted by $-OH$, $-NH_2$ or $C_1$-$C_4$-alkyl, $R^4$ is hydrogen, $C_1$-$C_{24}$-alkyl, a group $-X-OH$ or $-X-OCO-CH_2-$-$CO-R$, and $R^5$ is hydrogen, $-OH$, $-OR^3$, $-SR^3$, $-R^3$, $-NHR^3$ or $-X-OH$, Q is a group $-Z-CO$, wherein the carbonyl group is attached to the nitrogen of the formula II, and Z is ethylene which can be added to a 1,3-homodiene or (oxa- or aza-) heterodiene, which in turn can be the constituent, or partial constituent, of a homocyclic or (oxa-, thia- or aza-) heterocyclic ring system containing 5 to 40 ring members and 1 to 10 rings, or Z is vinylene, o-phenylene which can be substituted by a group $-CON(R^6)R^7$, $-COOR^8$ or $-COSR^8$, or by 4 halogen atoms, or is the group $-CH_2-S-CH_2-$, $R^6$ and $R^7$ are the same or different and are hydrogen or $C_1$-$C_{12}$-alkyl, and $R^8$ is $C_1$-$C_{12}$-alkyl or a group $-CH_2-COOR^9$, wherein $R^9$ is $C_1$-$C_{12}$-alkyl, or Q is a group which completes a 5- to 10-membered heterocyclic ring system containing at least 2 hetero atoms, and, if n is 2, A is one of the groups of the formulae:

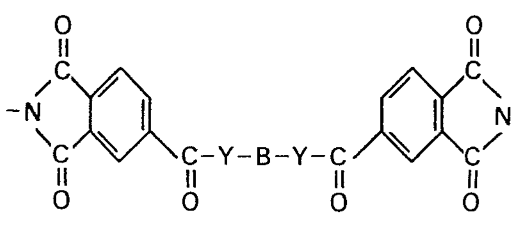

(VII)

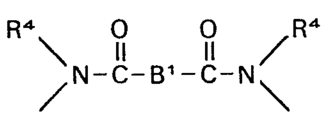

(VIII)

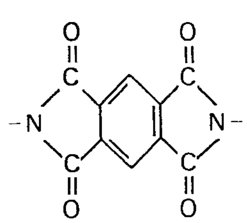

(IX)

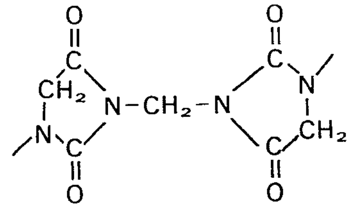

(X)

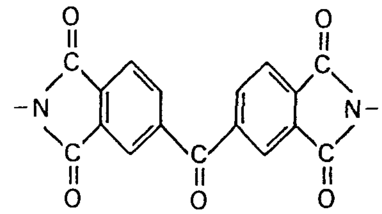

(XI)

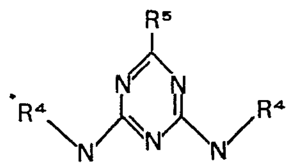

(XII)

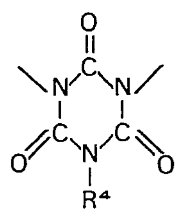

(XIII)

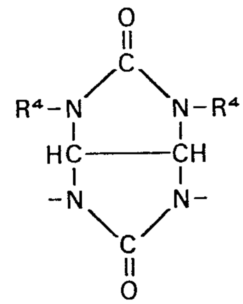

(XIV)

wherein Y can be $-O-$ or $-NH-$, B is $C_1$-$C_6$-alkylene, p-phenylene or one of the groups $[-(CH_2)_q-O]_r-(CH_2)_q$ or $[-(CH_2)_q-S]_r-(CH_2)_q$, wherein q is an integer from 1 to 4 and r is an integer from 1 to 3, and $B^1$ is a direct bond or has the same meaning as B, or is $C_{10}$-$C_{14}$-arylene or $C_5$-$C_8$-cycloalkylene, and, if n is 3, A is a group of the formula:

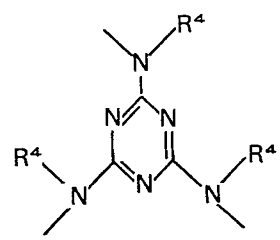

(XV)

wherein $R^4$ is as defined above.

2. A compound according to claim 1 of the formula I, wherein A is a group of the formula II and Q is one of the groups $-C(R^1)(R^2)-CO-N(R^4)-$, $-CO-C(R^1)(R^2)-CO-N(R^4)-$, $CR^1=CR^2-CO-N(R^4)-$, or:

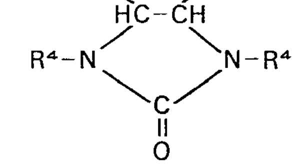

wherein each nitrogen is attached to the carbonyl group of the formula II, and $R^4$ is as defined in claim 1.

3. A compound according to claim 1 of the formula I, wherein n is 1 or 2, R is methyl or phenyl, $R^1$ and $R^2$ are hydrogen, and X is $C_2$-$C_3$-alkylene or a group $-CH_2CH_2O-CH_2CH_2-$, and, if n is 1, A is one of the groups of the formulae II, III, IV or V, wherein $R^3$ is $C_1$-$C_{24}$-alkyl, $R^4$ is a group $-X-OCO-CH_2-CO-R$, wherein X and R are as defined herein, and $R^5$ is $C_1$-$C_4$-alkoxy, and, if n is 2, A is one of the groups of the formulae VIII, IX, XI or XIV, wherein $B^1$ is a p-phenylene group and $R^4$ is hydrogen or the group $-X-OCO-CH_2-CO-R$, wherein X and R are as defined herein.

4. A compound according to claim 1 of the formula I, wherein R is methyl and X is $C_2$-$C_3$-alkylene, n is 1 or 2, and, if n is 1, A is a group of the formula:

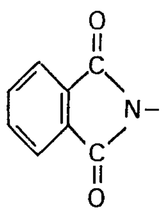

and, if n is 2, A is one of the groups of the formulae VIII, IX, XI or XIV, wherein B' is a p-phenylene group and $R^4$ is hydrogen or the group $-X-OCO-CH_2-CO-R$, wherein X and R are as defined herein.

5. A stabilised composition containing a chlorinated thermoplastic polymer and, as stabiliser, a compound, or a mixture of compounds, according to claim 1, in an amount of 0.05 to 5% by weight, based on the entire composition.

6. A stabilised composition according to claim 5 which additionally contains other stabilisers.

7. A stabilised composition according to claim 6, wherein one of the additional stabilisers is calcium/zinc carboxylate.

8. A stabilised composition according to claim 6, wherein one of the additional stabilisers is a phosphite.

9. A stabilised composition according to claim 5, wherein the chlorinated thermoplastic is a polymer of, or based on, vinyl chloride.

10. Use of a compound according to claim 1 for stabilising chlorinated thermoplastic polymers.

## Revendications

1. Composés répondant à la formule (I):

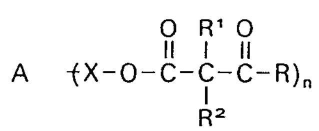 (I)

dans laquelle n désigne un nombre de 1 à 3, R représente un alkyle en $C_1$-$C_{24}$ou un phényle, $R^1$ et $R^2$ représentent chacun, indépendamment l'un de l'autre, l'hydrogène ou un radical tel que défini pour R, X représente un alkylène $C_1$-$C_6$ éventuellement interrompu par $-O-$, et A a, suivant la valeur de n, les significations suivantes:

— lorsque n est égal à 1, A représente un radical répondant à l'une des formules:

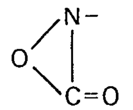 (II)

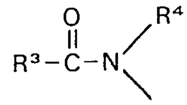 (III)

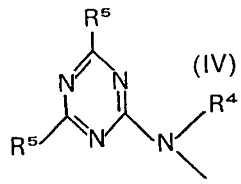 (IV)

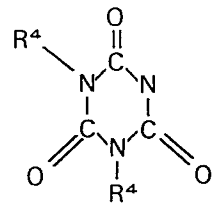 (V)

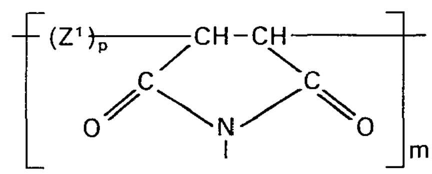 (VI)

dans lesquelles p est égal à 0 ou à 1, m désigne un nombre de 1 à 250, $Z^1$ représente un radical éthylène éventuellement porteur d'un alkyle en $C_1$-$C_{24}$, d'un alcoxy en $C_1$-$C_{24}$, d'un alcényle en $C_2$-$C_{24}$, d'un phényle, d'un alcoxycarbonyle en $C_2$-$C_{24}$, d'un phénoxycarbonyle, d'un alcanoyloxy en $C_2$-$C_{24}$, d'un benzyloxy, d'un halogène ou d'un groupe cyano, $R^3$ représente H, un alkyle en $C_1$-$C_{24}$ éventuellement porteur d'atomes d'halogènes, ou un radical aryle en $C_6$-$C_{14}$ ou aralkyle en $C_7$-$C_{16}$ éventuellement porteur d'un groupe $-OH$ ou $-NH_2$ ou d'un alkyle en $C_1$-$C_4$, $R^4$ représente l'hydrogène, un alkyle en $C_1$-$C_{24}$, un radical $-X-OH$ ou un radical $-X-OCO-CH_2-CO-R$, $R^5$ représente l'hydrogène ou un radical $-OH$, $-OR^3$, $-SR^3$, $-R^3$, $-NHR^3$ ou $-X-OH$, et Q représente un radical $-Z-CO-$ dont le groupe carbonyle est relié à l'azote présent dans la formule (II) et dont le symbole Z représente soit un radical éthylène, éventuellement fixé sur un homodiène-1,3 ou un hétérodiène-1,3 (oxa, aza), lequel peut, éventuellement partiellement, être un constituant d'un homocycle ou d'un hétérocycle (oxa, thia, aza) comportant de 5 à 40 maillons et de 1 à 10 noyaux, soit un radical vinylène, soit un radical o-phénylène éventuellement porteur d'un radical $-CON(R^6)R^7$, $-COOR^8$, $-COSR^8$ ou éventuellement de 4 atomes d'halogènes, soit un radical $-CH_2-S-CH_2-$, $R^6$ et $R^7$ représentant chacun, indépendamment l'un de l'autre, l'hydrogène ou un alkyle en $C_1$-$C_{12}$ et $R^8$ représentant un alkyle en $C_1$-$C_{12}$ ou un radical $-CH_2-COOR^9$ dans lequel $R^9$ représente un alkyle en $C_1$-$C_{12}$, ou Q représente un radical fermant le cycle qui conduit à un hétérocycle comportant de 5 à 10 maillons et contenant au moins 2 hétéroatomes,

— lorsque n est égal à 2, A représente un radical répondant à l'une des formules suivantes:

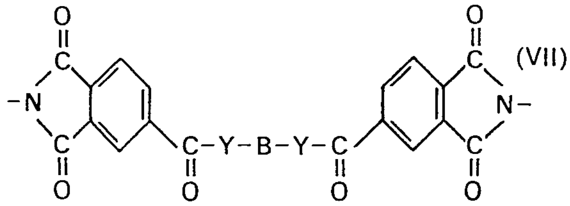

(VII)

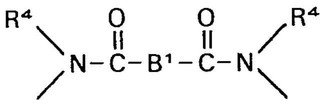

(VIII)

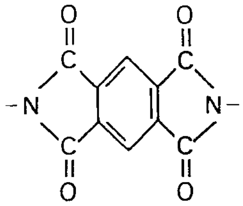

(IX)

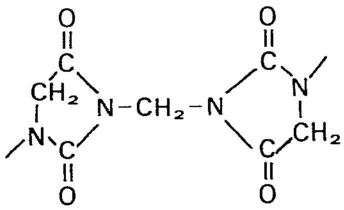

(X)

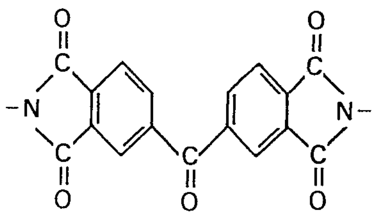

(XI)

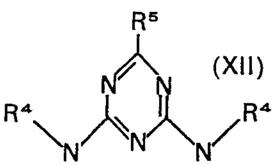

(XII)

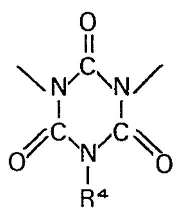

(XIII)

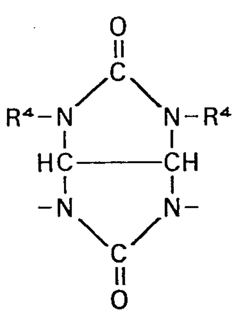

(XIV)

dans lesquelles Y représente $-O-$ ou $-NH-$, B représente un alkylène en $C_1$-$C_6$, un p-phénylène ou l'un des radicaux $[-(CH_2)_q-O]_r-(CH_2)_q$ et $[-(CH_2)_q-S-]_r-(CH_2)_q$ dans lesquels q désigne un nombre de 1 à 4 et r un nombre de 1 à 3, $B^1$ représente une liaison directe, l'un des radicaux mentionnés pour B, un arylène en $C_{10}$-$C_{14}$ ou un cycloalkylène en $C_5$-$C_8$, et

— lorsque n est égal à 3, A représente un radical répondant à la formule:

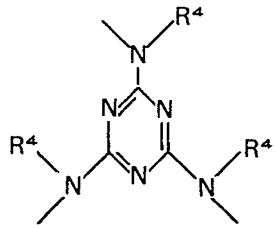

(XV)

dans laquelle $R^4$ a la signification qui a été donnée ci-dessus.

2. Composés de formule (I) selon la revendication 1, dans lesquels A représente un radical de formule (II) et Q représente un radical répondant à l'une des formules:
$-C(R^1)(R^2)-CO-N(R^4)-$, $-CO-C(R^1)(R^2)-CO-N(R^4)-$,
$-CR^1=CR^2-CO-N(R^4)-$ et

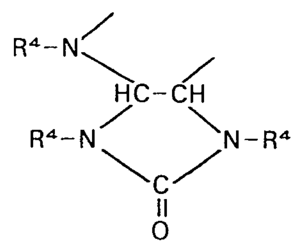

dans lesquelles $R^4$ a la signification donnée à la revendication 1, l'azote de ce radical Q étant à chaque fois relié au radical carbonyle présent dans la formule (II).

3. Composés de formule (I) selon la revendication 1, dans lesquels n désigne le nombre 1 ou 2, R représente un radical méthyle ou phényle, $R^1$ et $R^2$ représentent chacun l'hydrogène, X représente un alkylène en $C_2$ ou $C_3$ ou un radical $-CH_2CH_2-O-CH_2-CH_2-$, et, lorsque n est égal à 1, A représente l'un des radicaux de formules (II), (III), (IV) et (V) dans lesquels $R^3$ représente un alkyle en $C_1$-$C_{24}$, $R^4$ un radical $-X-OCO--CH_2-CO-R$ (dans lequel X et R ont les significations données à la revendication 1) et $R^5$ représente un alcoxy en $C_1$-$C_4$, et, lorsque n est égal à 2, A représente l'un des radicaux de formules (VIII), (IX) et (XIV) où $B^1$ représente un radical p-phénylène et $R^4$ représente l'hydrogène ou un radical $-X-OCO-CH_2-CO-R$ (dans lequel X et R ont les significations déjà données dans cette revendication).

4. Composés de formule (I) selon la revendication 1, dans lesquels R représente un radical méthyle, X représente un radical alkylène en $C_2$ ou $C_3$, n désigne le nombre 1 ou le nombre 2, et, lorsque n est égal à 1, A représente un radical de formule:

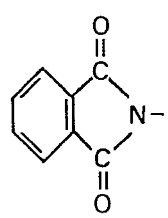

et, lorsque n est égal à 2, A représente l'un des radicaux de formule (VIII), (IX), (XI) et (XIV) où B' représente un radical p-phénylène et $R_4$

représente l'hydrogène ou un radical $-X-OCO-CH_2-CO-R$ (dans lequel X et R ont les significations déjà données pour ce cas préféré).

5. Composition stabilisée qui contient un polymère chloré thermoplastique et, comme stabilisant, un composé ou un mélange de composés selon la revendication 1, en une quantité de 0,05 à 5,0% en poids par rapport à la composition totale.

6. Composition stabilisée selon la revendication 5, caractérisée en ce qu'elle contient des stabilisants supplémentaires.

7. Composition stabilisée selon la revendication 6, caractérisée en ce que l'un des stabilisants supplémentaires est un carboxylate de Ca/Zn.

8. Composition stabilisée selon la revendication 6, caractérisée en ce que l'un des stabilisants supplémentaires est un phosphite.

9. Composition stabilisée selon la revendication 5, caractérisée en ce que la matière thermoplastique chlorée est un polymère du chlorure de vinyle ou à base de chlorure de vinyle.

10. Application d'un composé selon la revendication 1, pour la stabilisation de matières thermoplastiques contenant du chlore.